# EUROPEAN PATENT APPLICATION

(11) **EP 4 737 587 A1**
(43) Date of publication of application: **06.05.2026**
(21) Application number: 23942939.2
(22) Date of filing: 30.06.2023
(51) Int. Cl.: C12Q 1/6869, C12N 15/11

(54) **NANOPORE SEQUENCING METHOD USING CLOSER**

(71) Applicant: BGI Hangzhou CycloneSEQ Technoloy Co., Ltd., Hangzhou, Zhejiang 310030 (CN)
(72) Inventor: CHEN, Junyi, Shenzhen, Guangdong 518083 (CN); JI, Zhouxiang, Shenzhen, Guangdong 518083 (CN); ZENG, Tao, Shenzhen, Guangdong 518083 (CN); LI, Yuxiang, Shenzhen, Guangdong 518083 (CN); DONG, Yuliang, Shenzhen, Guangdong 518083 (CN); ZHANG, Wenwei, Shenzhen, Guangdong 518083 (CN); XU, Xun, Shenzhen, Guangdong 518083 (CN)
(74) Representative: Schwarz & Partner Patentanwälte GmbH
(86) International application number: PCT/CN2023/104486
(87) International publication number: WO 2025/000432

(57) **Abstract**

Provided in the present invention is a nanopore sequencing method using a closer. The method of the present invention enables a sequencing library to only bind within the nanopore capture range, ensuring that the library is efficiently captured by the nanopores during sequencing, thus improving the utilization efficiency of the library.

## Description

### FIELD

The present disclosure relates to the technical field of nanopore sequencing, and specifically to a nanopore sequencing method using a blocker.

### BACKGROUND

Nanopore sequencing is a single-molecule detection technique featuring advantages such as high sequencing speed, long read length, direct sequencing capability, high throughput, low cost, small size and portability. During nanopore sequencing, a single nanopore is embedded in an insulating and impermeable membrane to form a stable ionic current channel. The sequencing library is coupled to the insulating and impermeable membrane under the action of a tethering sequence. Driven by voltage, the sequencing library within a nanopore capture range translocates through the nanopore in the form of single-stranded nucleic acid molecules under the control of motor proteins, reducing the ionic current flowing through the nanopore. Due to the differences in molecular structure and volume among different bases on the single-stranded nucleic acid molecule, the current flowing through the nanopore exhibits variations corresponding to the base sequence. Using algorithms to analyze the current variation signals, the sequence of the translocated single-stranded nucleic acid can be read in real time.

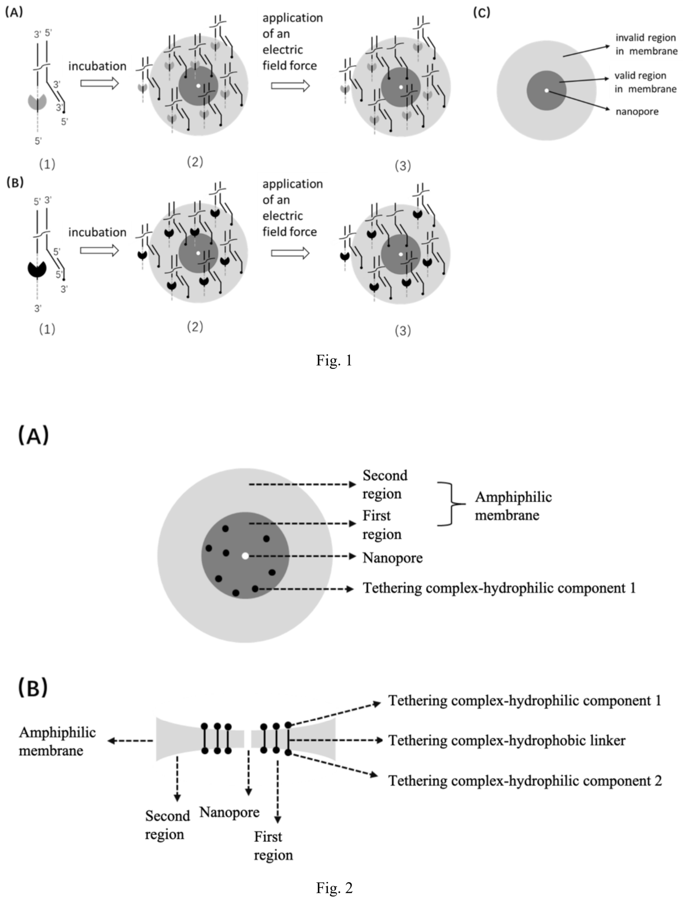

As developed by Oxford Nanopore Technologies (ONT), the library to be sequenced is coupled to the insulating and impermeable membrane under the action of a tethering sequence (Fig. 1), and the specific process is as follows: i. the library to be sequenced binds to the tethering sequence; ii. after incubation, the library to be sequenced is coupled to the insulating and impermeable membrane embedded with nanopores under the action of the tethering sequence; iii. upon application of an electric field force, the library to be sequenced coupled within a nanopore capture range is captured for sequencing (see Patent CN103733063B). However, when coupling to the insulating and impermeable membrane embedded with nanopores under the action of the tethering sequence, the sequencing library will also bind to such areas in the insulating and impermeable membrane that nanopores are not successfully embedded, or even to residual membrane materials in the sequencing system. However, only a small fraction of the sequencing library that is bound within the nanopore capture range can be captured by the nanopores and sequenced, leading to substantial waste of the library due to its failed sequencing. Developing a method enabling the sequencing library to be bound within the nanopore capture range in a concentrated manner can ensure efficient capture to the library by nanopores during sequencing and improve the utilization efficiency of the library.

A method developed by ONT for concentrating tethering complexes in an amphiphilic membrane region is illustrated in Fig. 2. The amphiphilic membrane is composed of multiple amphiphilic molecules and can be chemically and/or physically divided into a first region and a second region. Detectors, such as nanopores, are embedded in the first region. The tethering complex contains one or more hydrophilic components connected by a hydrophobic linker, and the tethering complex preferentially localises to the first region. The sequencing library can specifically bind to the hydrophilic components of the tethering complex, thereby binding the sequencing library to the first region (see Patent application CN115004030A). Qitan Technology Co., Ltd. Beijing has also developed a similar method (see Patent CN113999291B).

However, the method developed by ONT for concentrating tethering complexes in the amphiphilic membrane region includes the following drawbacks: i. although the amphiphilic membrane is chemically and/or physically divided into the first region and the second region, and the tethering complex preferentially localises to the first region, the first region does not necessarily completely overlap with the nanopore capture range, there is a portion of the sequencing library bound to the tethering complex still failing to be sequenced thus being wasted; ii. in a large-scale array sequencing system developed by ONT, not all amphiphilic membranes are embedded with nanopores capable of valid sequencing, or the nanopores that were initially functional may lose their activity after a period of sequencing; nevertheless, such amphiphilic membranes are still positioned with the tethering complexes in the first region, resulting in the waste of sequencing libraries bound to these tethering complexes due to failed sequencing; iii. the tethering complex is composed of one or more hydrophilic components connected by a hydrophobic linker, featuring a complex structure, which leads to high difficulty and cost in synthesis and purification; iv. a large number of hydrophobic linkers of the tethering complexes penetrate the amphiphilic membrane, which reduces the stability of the amphiphilic membrane and negatively affects the service life of the sequencing system; and v. it is required that the amphiphilic membrane and the tethering complex used in this method must be mutually compatible, resulting in poor universality. Similar drawbacks also exist in the similar method developed by Qitan Technology Co., Ltd Beijing.

Therefore, there is an urgent need in the art for a method to concentrate libraries to be sequenced to be bound within the nanopore capture range.

### SUMMARY

As described above, there is an urgent need in the art for a method to concentrate libraries to be sequenced to be bound within the nanopore capture range.

In a first aspect, there is provided in the present disclosure a nanopore sequencing method, including the following steps:
i. incubating a blocker with a membrane embedded with nanopores, such that the blocker is coupled to the membrane, wherein the blocker includes:
   a tethering sequence, including a portion coupled to the membrane;
   a first strand, including a first sequence, and a second sequence at least partially complementary to a second strand; and
   the second strand, including a third sequence at least partially complementary to the first strand, configured to bind with a sequencing library by means of complementation with a sequencing adapter, and a fourth sequence at least partially complementary to the tethering sequence,
ii. applying an electric field force to the membrane of step i, such that the first strand of the blocker within a nanopore capture range translocates through the nanopore, exposing the third sequence in the second strand of the blocker; and
iii. binding, the third sequence, with the sequencing library via the sequencing adapter, such that the sequencing library is bound within the nanopore capture range and further captured by the nanopore, thereby achieving a sequencing on a strand to be sequenced ligated with the sequencing adapter.

In a second aspect, there is provided in the present disclosure a nanopore sequencing kit, including a blocker, a sequencing buffer, a sequencing adapter and a second nucleic-acid-controlling-protein, wherein the blocker includes:
a tethering sequence, including a portion coupled to a membrane in the sequencing;
a first strand, including a first sequence, and a second sequence at least partially complementary to a second strand; and
the second strand, including a third sequence at least partially complementary to the first strand, and a fourth sequence at least partially complementary to the tethering sequence,
wherein the sequencing adapter forms, with the second nucleic-acid-controlling-protein, a sequencing adapter complex to capture a strand to be sequenced, thereby producing a sequencing library, and
the third sequence is configured to bind with the sequencing library complementarily, such that the sequencing library is bound within a nanopore capture range and further captured by the nanopore, to achieve sequencing.

The present disclosure is of the beneficial effects including:
i. the present disclosure ingeniously designs a blocker complementary to the adapter of the sequencing library, enabling the sequencing library to be bound only within the nanopore capture range, which ensures efficient capture to the library by nanopores during sequencing and improve the utilization efficiency of the library;
ii. the solution of the present disclosure allows the library introduced into the sequencing system but not sequenced after a single round of sequencing to suspend in the sequencing buffer; such that by directly aspirating the sequencing buffer from the sequencing system, almost all the unsequenced library can be recovered and reused;
iii. the blocker designed in the present disclosure has a simple structure and low cost;
iv. the blocker designed in the present disclosure exerts little impact on the stability of the membrane;
v. the blocker designed in the present disclosure has high universality for different types of insulating and impermeable membranes; and
vi. the translocation duration of a single blocker of the present disclosure is on the millisecond scale, which does not interfere with the capture of the sequencing library by the nanopores.

### BRIEF DESCRIPTION OF THE DRAWINGS

A brief description to the accompanying drawings will be made for illustrating the background of the present disclosure and the technical solutions set forth in the detailed description with greater clarity.
Fig. 1 is a schematic diagram illustrating a library to be sequenced coupled to an insulating and impermeable membrane embedded with nanopores, which is developed by Oxford Nanopore Technologies. In panel A, a helicase acts in a direction from 5' to 3'; while in panel B, a helicase acts in a direction from 3' to 5'. Panel C is a structure diagram for the membrane.
Fig. 2 is a schematic diagram illustrating a method for concentrating tethering complexes in an amphiphilic membrane region developed by Oxford Nanopore Technologies. Panel A shows a top view of the amphiphilic membrane, and panel B shows a cross-sectional view of the amphiphilic membrane.
Fig. 3 is a schematic diagram illustrating a method for concentrating a library to be sequenced to be bound within nanopore capture range through a blocker complex, according to embodiments of the present disclosure.
Fig. 4 is a schematic diagram illustrating a method for concentrating a library to be sequenced to be bound within nanopore capture range through a blocker, according to embodiments of the present disclosure.
Fig. 5 is a schematic diagram illustrating a construction of a blocker complex according to Example 1 of the present disclosure.
Fig. 6 shows electrophoretic results for blockers (A) and blocker complexes (B) after annealing according to Example 1 of the present disclosure.
Fig. 7 is a schematic diagram illustrating a construction of a sequencing adapter according to Example 1 of the present disclosure.
Fig. 8 shows electrophoretic results for sequencing adapters (A) and sequencing adapter complexes (B) after annealing according to Example 1 of the present disclosure.
Fig. 9 shows current signal profiles of three blocker complexes, blocker-10 iSpC3 (A), blocker-20 iSpC3 (B), and blocker-30 iSpC3 (C) translocating through the nanopore according to Example 1 of the present disclosure.
Fig. 10 shows a current signal profile during nanopore sequencing on a library-to-be-sequenced-1, using a blocker complex blocker-30 iSpC3 and a tethering-sequence-1, according to Example 1 of the present disclosure.
Fig. 11 is a schematic diagram illustrating a construction of a blocker according to Example 2 of the present disclosure.
Fig. 12 shows electrophoretic results for blockers according to Example 2 of the present disclosure.
Fig. 13 is a schematic diagram illustrating a construction of a sequencing adapter according to Example 2 of the present disclosure.
Fig. 14 shows electrophoretic results for sequencing adapters (A) and sequencing adapter complexes (B) after annealing according to Example 2 of the present disclosure.
Fig. 15 shows current signal profiles of three blockers, blocker-10 (A), blocker-20 (B), and blocker-30 (C) translocating through the nanopore according to Example 2 of the present disclosure.
Fig. 16 shows a current signal profile during nanopore sequencing on a library-to-be-sequenced-2, using a blocker blocker-20 and a tethering-sequence-2, according to Example 2 of the present disclosure.
Fig. 17 is a schematic diagram illustrating experimental designs for an experimental group using blocker-20 and tethering-sequence-2 (panel A), a control group 1 using tethering-sequence-1 (panel B) and a control group 2 using tethering-sequence-2 (panel C), according to Example 3 of the present disclosure.
Fig. 18 shows read capture yields in two replications of the experimental group, control group 1 and control group 2 each sequencing on a library-to-be-sequenced-2 at 50 ng according to Example 3 of the present disclosure, where A and B each correspond to one repetition.
Fig. 19 shows read capture yields in two replications of the experimental group, control group 1 and control group 2 each sequencing on a library-to-be-sequenced-2 at 5 ng according to Example 3 of the present disclosure, where A and B each correspond to one repetition.

### DETAILED DESCRIPTION

Reference will now be made in detail to embodiments, examples of which of the present disclosure are illustrated in the accompanying drawings. It should be understood that the embodiments described herein are explanatory, illustrative, and shall not be construed to limit the present disclosure. The scope of protection for the present disclosure shall be defined solely by the appended claims. Those skilled in the art will appreciate that modifications may be made to the technical solutions described herein without departing from the spirit and scope of the disclosure. Unless otherwise specified, the technical means employed in the embodiments are conventional means well known to a person skilled in the art.

Unless otherwise specified, all technical and scientific terms used herein shall be accorded the meanings commonly understood by one of ordinary skill in the art to which the present disclosure pertains. Hereafter, definitions are provided to facilitate a proper understanding to the present disclosure, prior to the detailed description of the disclosure.

In any instance where a numerical range is recited herein-including, but not limited to, ranges of concentration, percentage, or ratio-it is to be expressly construed that every numerical value falling between the stated upper and lower limits of said range, including each intervening value down to one-tenth of the unit of the lower limit, as well as any other recited value or any intermediate value within said range, is expressly encompassed within the scope of the disclosure. Any and all sub-ranges falling within a broader recited range are likewise encompassed, subject only to the exclusion of any specifically disclaimed endpoint within said broader range. Moreover, even where a recited range is expressed as including one or both of its endpoints, ranges expressly excluding either or both of said endpoints are also deemed to be within the scope of the disclosure.

Throughout the specification and claims, various embodiments are described using terminology of "comprising," "including," "containing," or "consisting essentially/mainly of". Unless the context clearly requires otherwise, these terms are to be interpreted in their open-ended sense, including, in addition to the element, component, step, or group thereof as recited in these terms, one or more other elements, components, steps, or groups. Conversely, it is to be understood that in specific contexts, any of said terms may be interpreted in a closed sense, synonymous with "consisting of," only concerning the element, component, step, or group thereof as recited in these terms, but expressly excluding any element, step, or ingredient not specified.

For the purpose of aiding in the understanding of the present disclosure without imposing undue limitations thereon, unless the context dictates otherwise, every numerical quantity, percentage, proportion, or other parameter set forth herein-whether in the specification or in the claims-is preceded by the modifier "about." Consequently, and unless expressly stated to the contrary, all numerical parameters set forth in this specification and the appended claims are to be regarded as approximations that may vary depending upon the properties sought to be achieved. At a minimum, each such numerical parameter shall be interpreted in light of the number of significant digits reported and by the application of standard rounding conventions.

As used herein, the term "nucleotide" refers to ribonucleotides, deoxyribonucleotides, or modified forms of either type of nucleotide, and combinations thereof; nucleotides are the basic structural units of nucleic acids.

As used herein, the term "nucleic acid" refers to a covalently connected sequence of nucleotides, either single-stranded or double-stranded, in which the 3' and 5' ends of each nucleotide are connected by phosphodiester bonds. The nucleic acid may be composed of deoxyribonucleotides or ribonucleotides. The nucleic acid may include DNA and RNA, and may be synthetically prepared in vitro or isolated from natural resources. Nucleic acids may further include modified DNA or RNA, such as methylated DNA or RNA, or post-transcriptionally modified RNA, such as 5'-capping with 7-methylguanosine, 3'-end processing (e.g., cleavage and polyadenylation), and splicing. Nucleic acids may also include xeno nucleic acids (XNA), such as hexitol nucleic acid (HNA), cyclohexenyl nucleic acid (CeNA), threose nucleic acid (TNA), glycerol nucleic acid (GNA), bridged nucleic acid (BNA), locked nucleic acid (LNA), and peptide nucleic acid (PNA). The length of a nucleic acid is typically represented with the number of base pairs (bp) for double-stranded polynucleotides, or the number of nucleotides (nt) for single-stranded polynucleotides.

As used herein, the term "nanopore" refers to a structure having a nanoscale channel through which an ionic current is flowable. An inner diameter of a nanopore may vary greatly depending on the intended use of a device.

As used herein, the term "nucleic-acid-controlling-protein" refers to a class of proteins that can bind to a single-stranded nucleotide of a nucleotide chain and perform functions such as polymerization, unwinding, or ligation of the nucleotide chain. For example, the nucleic-acid-controlling-protein may be a helicase, topoisomerase, ligase, or polymerase. Typically, a nucleic-acid-controlling-protein may bind to a nucleic-acid-controlling-protein binding sequence of a blocker, or alternatively, to an adapter sequence of a sequencing library.

As used herein, the term "blocking sequence" refers to a class of compound molecules that can inhibit the nucleic-acid-controlling-proteins from performing functions such as polymerization, unwinding, or ligation of a nucleotide chain during nanopore sequencing. For example, when a blocker complex is not captured by a nanopore, the presence of the blocking sequence prevents a helicase from unwinding complementary sequences in the blocker; while the blocker complex is captured by the nanopore, the helicase moves across the blocking sequence in the blocker under the action of an electric field, and initiates the unwinding of the complementary sequences in the blocker. The molecular structure of the blocking sequence is not particularly limited; for instance, it may be iSp18, iSp9, iSpC3, iSpC6, iSpC12, or a modified oligonucleotide. Blocking sequences are generally commercially available, e.g., purchased from Sangon Biotech (Shanghai) Co., Ltd. (https://www.sangon.com/conventional_primer_synthesis.html).

As used herein, the term "tethering sequence" refers to a polymer sequence (e.g., a nucleic acid sequence) modified with a hydrophobic molecule at an end. The tethering sequence couples one end to a membrane via the hydrophobic molecule, and the other end, by complementary pairing with the sequencing adapter, draws the library ligating the sequencing adapter close to the membrane. The length of the tethering sequence is not particularly limited; for example, it may be 10 to 50 nucleotides. A portion of the tethering sequence may bind to the fourth sequence of the blocker.

As used herein, the term "guide sequence" refers to the first sequence in the first strand (i.e., the top strand) of a blocker, allowing the blocker to be captured by the nanopore, thereby enabling the first strand (i.e., the top strand) of the blocker to pass through the nanopore.

As used herein, the term "exposure/exposing/exposed" refers to a state exhibited by the third sequence of the second strand of the blocker during sequencing. Prior to translocation of the blocker through the nanopore, the third sequence of the second strand of the blocker is in a complementary binding state with the second sequence of the first strand. After the first strand of the blocker is captured by the nanopore and translocated through the same, the third sequence of the second strand is "exposed" in a single-stranded state, capable of binding to the sequencing adapter.

As used herein, relational terms such as first and second are used merely to distinguish one object or operation from another object or operation, and do not necessarily require or imply any actual such relationship or order between these objects or operations.

As described above, the present disclosure aims at providing a method to concentrate libraries to be sequenced to be bound within the nanopore capture range.

Accordingly, In a first aspect, there is provided in embodiments of the present disclosure a nanopore sequencing method, including the following steps:
i. incubating a blocker with a membrane embedded with nanopores, such that the blocker is coupled to the membrane, wherein the blocker includes:
   a tethering sequence, including a portion coupled to the membrane;
   a first strand (also referred to as "top strand"), including a first sequence (also referred to as "guide sequence"), and a second sequence at least partially complementary to a second strand; and
   the second strand (also referred to as "bottom strand"), including a third sequence at least partially complementary to the first strand, configured to bind with a sequencing library by means of complementation with a sequencing adapter, and a fourth sequence at least partially complementary to the tethering sequence,
ii. applying an electric field force to the membrane of step i, such that the first strand of the blocker within a nanopore capture range translocates through the nanopore, exposing the third sequence in the second strand of the blocker; and
iii. binding, the third sequence, with the sequencing library via the sequencing adapter, such that the sequencing library is bound within the nanopore capture range and further captured by the nanopore, thereby achieving a sequencing on a strand to be sequenced ligated with the sequencing adapter.

In the present disclosure, a sequencing library is bound within the nanopore capture range by binding the sequence adapter with the blocker sequence complementarily, and further captured by the nanopore, to achieve sequencing. Specifically, in one embodiment of the present disclosure, a strand to be sequenced is ligated with a sequencing adapter complex by means of a DNA ligase to construct a sequencing library, where the sequencing adapter complex includes a sequencing adapter and a second nucleic-acid-controlling-protein binding to the sequencing adapter, which has a similar structure to the blocker, including a top strand and a bottom strand. The top strand includes a guide sequence, a nucleic-acid-controlling-protein binding sequence, a blocking sequence, and a sequence complementary to the bottom strand, while the bottom strand includes a sequence complementary to the top strand, and a partial sequence of the top strand or the bottom strand may be complementarily bound to the third sequence in the blocker.

In an embodiment, the portion coupled to the membrane in the tethering sequence is a hydrophobic molecule. In a preferable embodiment, the hydrophobic molecule is selected from any one or more of a lipid, fatty acid, sterol, carbon nanotube, polypeptide, protein and/or amino acid. In a further preferable embodiment, the hydrophobic molecule is selected from any one or more of cholesterol, palmitate or tocopherol.

In an embodiment, the first sequence consists of 10 to 50 nucleotides, iSpC3, iSp18, or a combination thereof.

In an embodiment, the first strand further includes a nucleic-acid-controlling-protein binding sequence, and a first nucleic-acid-controlling-protein is introduced in step i, such that the first nucleic-acid-controlling-protein binds to the nucleic-acid-controlling-protein binding sequence. The first nucleic-acid-controlling-protein is selected from any one of a helicase, topoisomerase, ligase, or polymerase. The first nucleic-acid-controlling-protein acts in a direction from 3' to 5'or from 5' to 3'.

In a further preferable embodiment, the first strand further includes a blocking sequence, located between the second sequence and the nucleic-acid-controlling-protein binding sequence. The blocking sequence consists of at least one of iSp18, iSp9, iSpC3, iSpC6, and iSpC12. The blocking sequence has a length of at least 1 bp.

In an embodiment, the second sequence, the third sequence, and the fourth sequence each has a length of at least 2 bp. In a preferable embodiment, the second sequence, the third sequence, and the fourth sequence each has a length of 5 bp to 30 bp.

In an embodiment, the third sequence is complementary, in a length of at least 2 bp, to the sequencing adapter of the sequencing library.

In an embodiment, the blocker is composed of DNA, RNA, LNA, PNA, BNA, or any combination thereof.

In an embodiment, the nanopore is a transmembrane protein pore or a solid-state pore. In a preferable embodiment, the transmembrane protein pore is selected from hemolysin, MspA, MspB, MspC, MspD, FraC, ClyA, PA63, CsgG, CsgD, XcpQ, SP1, phi29 connector protein, InvG, GspD, or any combination thereof.

In an embodiment, the membrane is an amphiphilic membrane, a high-molecular polymer membrane, or any combination thereof.

In an embodiment, a voltage applied during sequencing is above 10 mV. In a preferable embodiment, the voltage is 50 mV to 250 mV.

In an embodiment, the sequencing library is introduced into a reaction system simultaneously with, prior to, or subsequent to an introduction of the blocker.

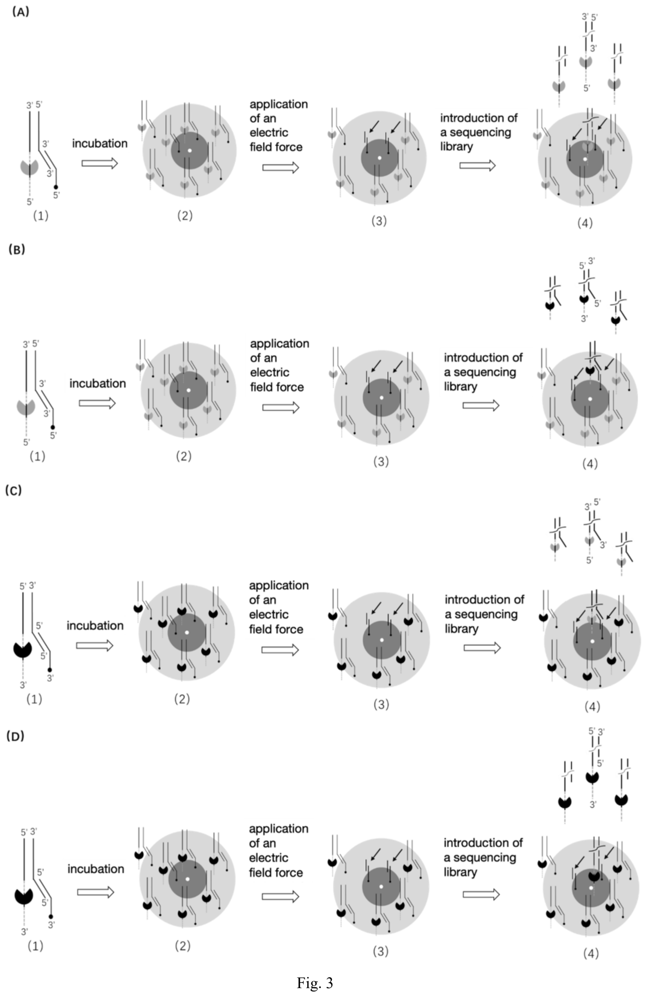

In an embodiment, the sequencing adapter consists of two nucleic acid chains, with a side of one nucleic acid chain complementary to a side of the other nucleic acid chain, and another side of one nucleic acid chain complementary to another side of the other nucleic acid chain, the blocker binds to the sequencing adapter in a manner includes:
a. under the sequencing adapter having a Y-shaped structure, with a side being a non-complementary, single stranded portion and another side being a complementary, double stranded portion, the third sequence of the blocker complementarily binds to the single stranded portion of the sequencing adapter (as shown in panels B and C of Fig. 3); or
b. under the sequencing adapter having a side being a non-complementary, single stranded portion and another side being a complementary, double stranded portion including a RNA fragment, after degrading the RNA fragment with a ribonuclease, the third sequence of the blocker complementarily binds to a complementary sequence of the RNA fragment (as shown in panels A and D of Fig. 3).

In an embodiment, the sequencing adapter binds with a second nucleic-acid-controlling-protein, forming a sequencing adapter complex, where the second nucleic-acid-controlling-protein is selected from any one of a helicase, topoisomerase, ligase, or polymerase. In a preferable embodiment, the second nucleic-acid-controlling-protein is a helicase.

In an embodiment, in step iii, the third sequence of the blocker is complementary to the sequencing adapter of the sequencing library, such that the blocker links to the sequencing library; and under an action of the electric field force, the strand to be sequenced in the sequencing library translocates through the nanopore, and a current signal change generated by translocation of the strand to be sequenced through the nanopore is detected, thereby obtaining sequence information of the strand to be sequenced.

In a second aspect, there is provided in embodiments of the present disclosure a nanopore sequencing kit, including a blocker, a sequencing buffer, a sequencing adapter and a second nucleic-acid-controlling-protein, wherein the blocker includes:
a tethering sequence, including a portion coupled to a membrane in the sequencing;
a first strand, including a first sequence, and a second sequence at least partially complementary to a second strand; and
the second strand, including a third sequence at least partially complementary to the first strand, and a fourth sequence at least partially complementary to the tethering sequence,
wherein the sequencing adapter forms, with the second nucleic-acid-controlling-protein, a sequencing adapter complex to capture a strand to be sequenced, thereby producing a sequencing library, and
the third sequence is configured to bind with the sequencing library complementarily, such that the sequencing library is bound within a nanopore capture range and further captured by the nanopore, to achieve sequencing.

In an embodiment, the portion coupled to the membrane in the tethering sequence is a hydrophobic molecule. In a preferable embodiment, the hydrophobic molecule is selected from any one or more of a lipid, fatty acid, sterol, carbon nanotube, polypeptide, protein and/or amino acid. In a further preferable embodiment, the hydrophobic molecule is selected from any one or more of cholesterol, palmitate or tocopherol.

In an embodiment, the first sequence consists of 10 to 50 nucleotides, iSpC3, iSp18, or a combination thereof.

In an embodiment, the first strand further includes a nucleic-acid-controlling-protein binding sequence, and the kit further includes a first nucleic-acid-controlling-protein, such that the first nucleic-acid-controlling-protein binds to the nucleic-acid-controlling-protein binding sequence. The first nucleic-acid-controlling-protein is selected from any one of a helicase, topoisomerase, ligase, or polymerase. The first nucleic-acid-controlling-protein acts in a direction from 3' to 5'or from 5' to 3'.

In a further preferable embodiment, the first strand further includes a blocking sequence, located between the second sequence and the nucleic-acid-controlling-protein binding sequence. The blocking sequence consists of at least one of iSp18, iSp9, iSpC3, iSpC6, and iSpC12. The blocking sequence has a length of at least 1 bp.

In an embodiment, the second sequence, the third sequence, and the fourth sequence each has a length of at least 2 bp. In a preferable embodiment, the second sequence, the third sequence, and the fourth sequence each has a length of 5 bp to 30 bp.

In an embodiment, the third sequence is complementary, in a length of at least 2 bp, to the sequencing adapter of the sequencing library.

In an embodiment, the blocker is composed of DNA, RNA, LNA, PNA, BNA, or any combination thereof.

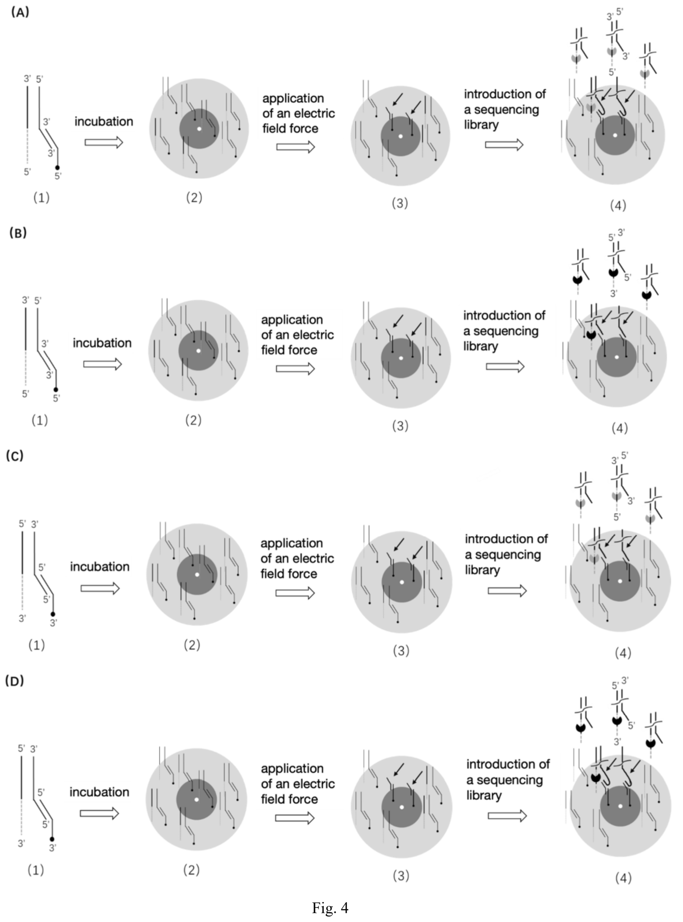

Fig. 3 and Fig. 4 respectively shows a method to concentrate libraries to be sequenced to be bound within the nanopore capture range using a blocker complex (i.e., a blocker and a nucleic-acid-controlling-protein) and using a blocker according to embodiments of the present disclosure. Details to the method are explained as follows.

Fig. 3 illustrates processes of nanopore sequencing using the blocker complex under the following four scenarios: A: blocker Complex 1 including a 5'→3' helicase + a 5'→3' tethering sequence + a library bound with a 5'→3' helicase; B: blocker Complex 1 including a 5'→3' helicase + a 5'→3' tethering sequence + a library bound with a 3'→5' helicase; C: blocker Complex 2 including a 3'→5' helicase + a 3'→5' tethering sequence + a library bound with a 5'→3' helicase; and D: blocker Complex 2 including a 3'→5' helicase + a 3'→5' tethering sequence + a library bound with a 3'→5' helicase.

The specific process is illustrated by taking panel A as an example.

At step (1): the blocker complex is prepared, in which the blocker complex includes a top strand, a bottom strand, a tethering sequence and a helicase. From the 5' to 3' end, the top strand sequentially includes a guide sequence (indicated by a gray dashed line), a helicase-binding sequence (indicated by a black full line), a blocking sequence (indicated by a gray dashed line), and a bottom strand-complementary sequence (indicated by a black full line). From the 5' to 3' end, the bottom strand sequentially includes a top strand-complementary sequence and a tethering sequence-complementary sequence (both indicated by black full lines). The blocker, with successful anneal, is incubated with a helicase with a helicase direction of 5' to 3' (represented by a light gray circle with a gap) and purified to form the blocker complex.

At step (2): after incubation, the blocker complex is coupled to an insulating and impermeable membrane under the action of the tethering sequence.

At step (3): upon application of an electric field force, the blocker complex bound within the nanopore capture range is captured; the top strand of the blocker complex translocates through the nanopore, while the bottom strand remains bound to the tethering sequence and exposes a sequence (indicated by arrows) that binds with a sequencing library. During this, due to the short length of the top strand of the blocker, the translocation duration of a single blocker complex is on the order of 100 milliseconds, which does not interfere with the capture of the sequencing library by the nanopore.

At step (4): the sequencing library is introduced, which could only bind to the bottom strand of the blocker that is located within the nanopore capture range and has the library-binding sequence exposed (indicated by arrows). This allows the library to be sequenced to be concentrated within the nanopore capture range, thereby ensuring efficient capture to the library by nanopores during sequencing and improve the utilization efficiency of the library.

Fig. 4 illustrates processes of nanopore sequencing using the blocker under the following four scenarios: A: blocker 1 + a 5'→3' tethering sequence + a library bound with a 5'→3' helicase; B: blocker + a 5'→3' tethering sequence + a library bound with a 3'-5' helicase; C: blocker 2 + a 3'→5' tethering sequence + a library bound with a 5'→3' helicase; and D: blocker 2 + a 3'→5' tethering sequence + a library bound with a 3'→5' helicase.

The specific process is illustrated by taking panel C as an example.

At step (1): the blocker is prepared, in which the blocker includes a top strand, a bottom strand, and a tethering sequence. From the 5' to 3' end, the top strand sequentially includes a bottom strand-complementary sequence (indicated by a black full line) and a guide sequence (indicated by a gray dashed line). From the 5' to 3' end, the bottom strand sequentially includes a tethering sequence-complementary sequence and a top strand-complementary sequence (both indicated by black full lines).

At step (2): after incubation, the blocker is coupled to an insulating and impermeable membrane under the action of the tethering sequence.

At step (3): upon application of an electric field force, the blocker bound within the nanopore capture range is captured; the top strand of the blocker translocates through the nanopore, while the bottom strand remains bound to the tethering sequence and exposes a sequence (indicated by arrows) that binds with a sequencing library. During this, due to the short length of the top strand of the blocker, the translocation duration of a single blocker is on the millisecond scale, which does not interfere with the capture of the sequencing library by the nanopore.

At step (4): the sequencing library is introduced, which could only bind to the bottom strand of the blocker that is located within the nanopore capture range and has the library-binding sequence exposed (indicated by arrows). This allows the library to be sequenced to be concentrated within the nanopore capture range, thereby ensuring efficient capture to the library by nanopores during sequencing and improve the utilization efficiency of the library.

The aforementioned sequencing employs the essential equipment and reagents required for sequencing, including a nanopore sequencer, a sequencing buffer and the like. The membrane of the nanopore sequencer is a phospholipid bilayer, a diblock copolymer, or a triblock copolymer.

The sequencing buffer contains a pH buffer system of a dihydrogen phosphate-hydrogen phosphate buffer system, a carbonic acid-sodium bicarbonate buffer system, a Tris-HCl buffer system, a HEPES buffer system, a MOPS buffer system, or any combination thereof.

The sequencing buffer contains NTPs, dNTPs, ddNTPs, or any combination thereof.

The sequencing buffer contains K⁺, Na⁺, or any combination thereof

The sequencing buffer contains Mg²⁺, Mo²⁺, Cu²⁺, Fe²⁺, Zn²⁺, Ca²⁺, Pb²⁺, Cd²⁺, or any combination thereof.

### Example

It should be noted that any terminology in Examples are explanatory, serve to explain the present disclosure, and are not construed to limit Examples of the present disclosure. The "Summary" section hereinabove and the detailed description hereinafter are provided solely for the purpose of explicating the disclosure and shall not be construed as imposing any limitation thereon. Without departing from the spirit and gist of the disclosure, the scope of the present disclosure shall be determined exclusively by the appended claims.

### Example 1

This Example was conducted with a blocker complex shown in panel A of Fig. 3 according to the present disclosure, specifically including the following steps.

### 1.1 Preparation of blocker Complex

The following sequences were separately dissolved with TE buffer (pH = 8) in accordance with the manufacturer's instructions.

5'-XXXXXXXXXXTTTTTTTTTTYYYYGGTTGTTTCTGTTGG-3' (X=iSpC3, Y=iSp18; SEQ ID NO: 1); 5'-XXXXXXXXXXXXXXXXXXXXTTTTTTTTTTYYYYGGTTGTTTCTGTTGG-3' (X=iSpC3 , Y=iSp18; SEQ ID NO: 2); 5'-XXXXXXXXXXXXXXXXXXXXXXXXXXXXXXTTTTTTTTTTYYYYGGTTGTTTCTGTTGG-3' (X=iSpC3, Y=iSp18; SEQ ID NO: 3); 5'-CCAACAGAAACAACCTTTGAGGCGAGCGGTCAA-3' (SEQ ID NO: 4).

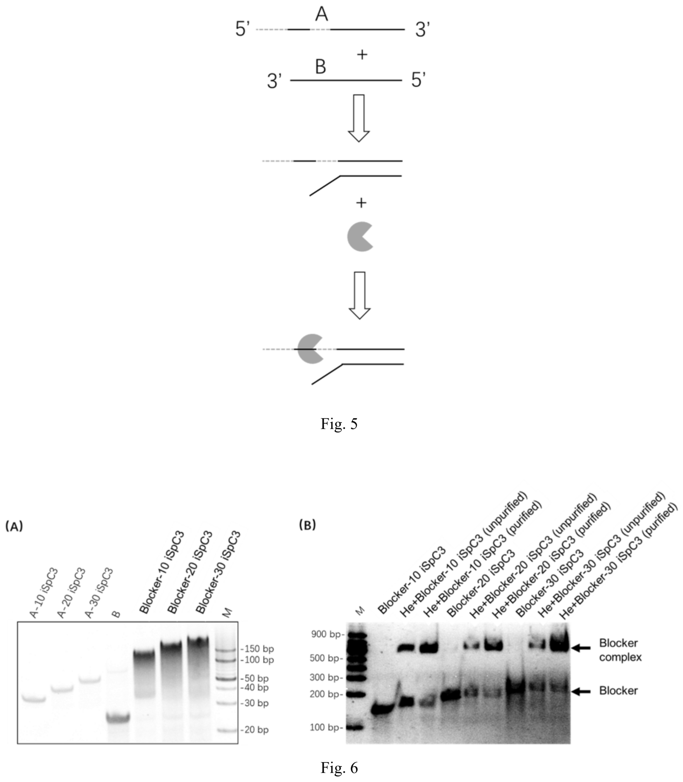

The top strands (SEQ ID NO: 1, SEQ ID NO: 2, SEQ ID NO: 3) were individually subject to anneal with the bottom strand (SEQ ID NO: 4) at a molar ratio of 1:1 to prepare blockers, which were named as blocker-10 iSP3, blocker-20 iSP3, and blocker-30 iSP3, respectively. The annealing procedure is as follows: incubate at 95 °C for 5 minutes, cool down to 25 °C at a rate of 0.1 °C/s, and continue incubation for 30 minutes. The structures of the top and bottom strands of the blocker are shown in Fig. 5, where the top strand A sequentially includes, in the 5' to 3' direction, a guide sequence (10 iSpC3, 20 iSpC3, or 30 iSpC3 units, indicated by gray dashed line), a helicase-binding sequence (10 thymine residues, indicated by black full line), a blocking sequence (4 iSp18 units, indicated by gray dashed line), and a bottom strand-complementary sequence (indicated by black full line); and the bottom strand B sequentially includes, in the 5' to 3' direction, a top strand-complementary sequence and a tethering-sequence-1-complementary sequence (both indicated by black full lines), where the tethering-sequence-1 is 5'-Cholesterol-YYYYTTGACCGCTCGCCTC-3' (Y=iSp18; SEQ ID NO: 5). After annealing, electrophoresis detection was performed. The results are shown in part A of Fig. 6, demonstrating that the annealed blockers were successfully prepared, where lanes 1-3 show the top strands A-10 iSpC3, A-20 iSpC3, A-30 iSpC3 (SEQ ID NOs: 1-3) respectively, lane 4 shows the bottom strand (B; SEQ ID NO: 4), lanes 5-7 show the annealed blocker-10 iSpC3, blocker-20 iSpC3, and blocker-30 iSpC3 respectively, and lane 8 shows the DNA molecular weight marker (M).

A helicase He (T4Dda-(ΔM1) G1/E94C/C109A/C136A/K194L/A360C; SEQ ID NO: 6) was expressed in prokaryotic cells *Escherichia coli,* and was obtained as a target protein through multi-step purification. The amino acid sequence of the helicase is as follows:

GSHGTFDDLTEGQKNAFNIVMKAIKEKKHHVTINGPAGTGKTTLTKFIIEALISTGETGIILA
APTHAAKKILSKLSGKEASTIHSILKINPVTYECNVLFEQKEVPDLAKARVLICDEVSMYDRKLF
KILLSTIPPWATIIGIGDNKQIRPVDPGENTAYISPFFTHKDFYQCELTEVKRSNAPIIDVATDVRNG
LWIYDKVVDGHGVRGFTGDTALRDFMVNYFSIVKSLDDLFENRVMAFTNKSVDKLNSIIRKKIF
ETDKDFIVGEIIVMQEPLFKTYKIDGKPVSEIIFNNGQLVRIIEAEYTSTFVKARGVPGEYLIRHWD
LTVETYGDDEYYREKIKIISSDEELYKFNLFLGKTCETYKNWNKGGKAPWSDFWDAKSQFSKV
KALPASTFHKAQGMSVDRAFIYTPCIHYADVELAQQLLYVGVTRGRYDVFYV (SEQ ID NO: 6).

The blocker and the heliccase (represented by the light gray circle with a gap in Fig. 5, with a helicase direction of 5' to 3') were incubated to form a complex. Specifically, the helicase He was mixed with the blocker-10 iSpC3, blocker-20 iSpC3, and blocker-30 iSpC3 each at a molar ratio of 9:1, in a reaction buffer with a final concentration of 25 mM HEPES, 50 mM KCl, 0.5 mM EDTA, and 2.5 mM MgCl₂ (pH = 8.0), and was incubated at room temperature for 30 minutes.

To the incubation product 0.25 volumes of 5 mM ATP was added, and was incubated at room temperature for 30 minutes. Owing to the blocking sequence, only 1 molecule of the helicase He bound between the guide sequence and the blocking sequence was retained.

The blocker complexes were purified with AMPure XP beads (Beckman Coulter, Cat. No. A63882), to obtain the target blocker complexes. The purified blocker complexes were subject to electrophoresis detection, and the results are shown in part B of Fig. 6, demonstrating that a large quantity of blocker complexes were successfully prepared, where lane 1 shows the DNA molecular weight marker (M), lanes 2-4 respectively shows the blocker-10 iSpC3, the unpurified complex obtained by the incubation of the blocker-10 iSpC3 with the helicase, and a purified one derived from the same incubation; lanes 5-7 respectively shows the blocker-20 iSpC3, the unpurified complex obtained by the incubation of the blocker-20 iSpC3 with the helicase, and a purified one derived from the same incubation; and lanes 8-10 respectively shows the blocker-30 iSpC3, the unpurified complex obtained by the incubation of the blocker-30 iSpC3 with the helicase, and a purified one derived from the same incubation.

### 1.2 Preparation of Sequencing Library

The following sequences were separately dissolved with TE buffer (pH = 8) in accordance with the manufacturer's instructions.

5'-XXXXXXXXXXXXXXXXXXXXXXXXXXXXXXTTTTTTTTTTYYYYGGTTGTTTCTGTTGGTG CTGATATTGCT-3' (X=iSpC3, Y=iSp18; SEQ ID NO: 7); 5'-Phosphorylation-GCAATATCA-3' (SEQ ID NO: 8); 5'-CCAACAGAAACAACC-3' (SEQ ID NO: 9, RNA).

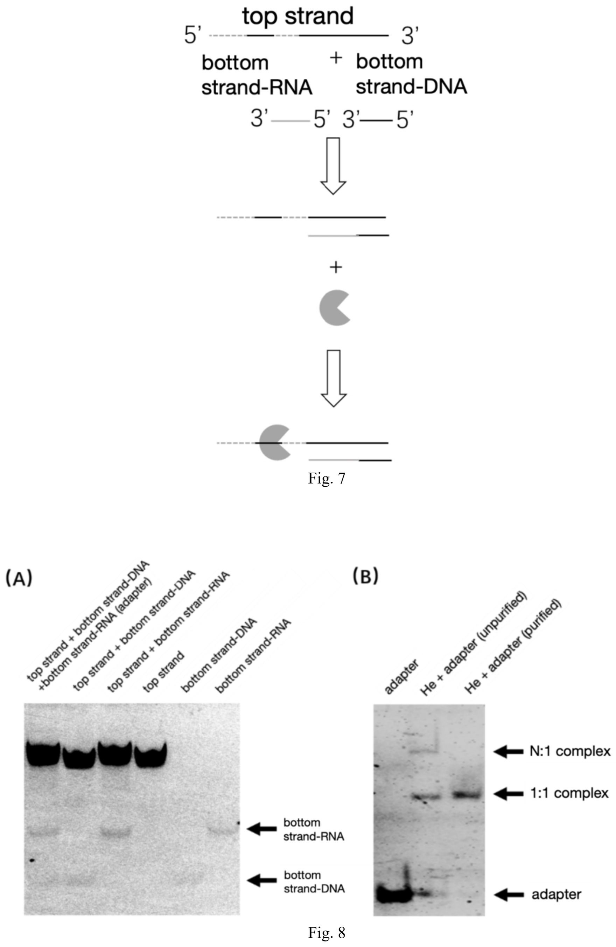

The sequences of SEQ ID NO: 7, SEQ ID NO: 8 and SEQ ID NO: 9 were subject to anneal at a molar ratio of 1:1:1 to prepare a sequencing adapter. The annealing procedure is as follows: incubate at 95 °C for 5 minutes, cool down to 25 °C at a rate of 0.1 °C/s, and continue incubation for 30 minutes. The sequences of "SEQ ID NO: 7 and SEQ ID NO: 9", and "SEQ ID NO: 7 and SEQ ID NO: 8" were each subject to anneal at a molar ratio of 1:1 for control. The structure of the sequencing adapter is shown in Fig. 7, where the top strand A sequentially includes, in the 5' to 3' direction, a guide sequence (30 iSpC3 units, indicated by gray dashed line), a helicase-binding sequence (10 thymine residues, indicated by black full line), a blocking sequence (4 iSp18 units, indicated by gray dashed line), and a bottom strand-complementary sequence (indicated by black full line); the bottom-strand-DNA is a top strand-complementary sequence (indicated by black full line); and the bottom-strand-RNA, adjacent to the bottom-strand-DNA, is a top strand-complementary sequence (indicated by gray dashed line).

After annealing, electrophoresis detection was performed. The results are shown in part A of Fig. 8, demonstrating that the sequences of SEQ ID NO: 7, SEQ ID NO: 8 and SEQ ID NO: 9 were successfully annealed at a ratio of 1:1:1 to obtain the sequencing adapter, where lane 1 shows the sequencing adapter obtained by anneal of the top strand (SEQ ID NO: 7), the bottom-strand-DNA (SEQ ID NO: 8) and the bottom-strand-RNA (SEQ ID NO: 9) at a ratio of 1:1:1; lane 2 shows a control obtained by anneal of the top strand and bottom-strand-DNA a ratio of 1:1; lane 3 shows a control obtained by anneal of the top strand and bottom-strand-RNA a ratio of 1:1; lane 4 shows the top strand; lane 5 shows the bottom-strand-DNA; and lane 6 shows the bottom-strand-RNA.

The helicase He (T4Dda-(ΔM1) G1/E94C/C109A/C136A/K194L/A360C; SEQ ID NO: 6) was expressed in prokaryotic cells *Escherichia coli,* and was obtained as a target protein through multi-step purification.

The heliccase and the sequencing adapter were mixed at a molar ratio of 9:1 in a reaction buffer with a final concentration of 25 mM HEPES, 50 mM KCl, 0.5 mM EDTA, and 2.5 mM MgCl₂ (pH = 8.0), and were incubated at room temperature for 30 minutes.

To the incubation product 0.25 volumes of 5 mM ATP was added, and was incubated at room temperature for 30 minutes. Owing to the blocking sequence, only 1 molecule of the helicase He bound between the guide sequence and the blocking sequence was retained.

The sequencing adapter complexes were purified with AMPure XP beads (Beckman Coulter, Cat. No. A63882), to obtain the purified sequencing adapter complexes (refer to Fig. 7, the helicase is represented by the light gray circle with a gap, with a helicase direction of 5' to 3'). The purified sequencing adapter complexes were subject to electrophoresis detection, and the results are shown in part B of Fig. 8, demonstrating that a large quantity of sequencing adapter complexes each with the sequencing adapter and the helicase at a ratio of 1:1, where lanes 1-3 respectively shows the sequencing adapter, the unpurified complex obtained by the incubation of the adapter with the helicase, and a purified one derived from the same incubation.

The sequencing adapter complex was ligated to randomly fragmented genomic DNAs of *Bacillus subtilis* by T4 DNA Ligase (NEB, Cat. No. M0202T). Subsequently, the RNA in the sequencing adapter was degraded with RNase H (NEB, Cat. No. M0297L), and with purification via AMPure XP beads, a library-to-be-sequenced-1 was obtained.

### 1.3 Nanopore Sequencing with the blocker Complex

In accordance with the reference (Ji Z, Guo P. Channel from bacterial virus T7 DNA packaging motor for the differentiation of peptides composed of a mixture of acidic and basic amino acids. Biomaterials. 2019 May 21; 214:119222), a single-channel nanopore detection system was constructed based on a patch-clamp and a signal amplifier, with an embedment of a single pore protein into the membrane.

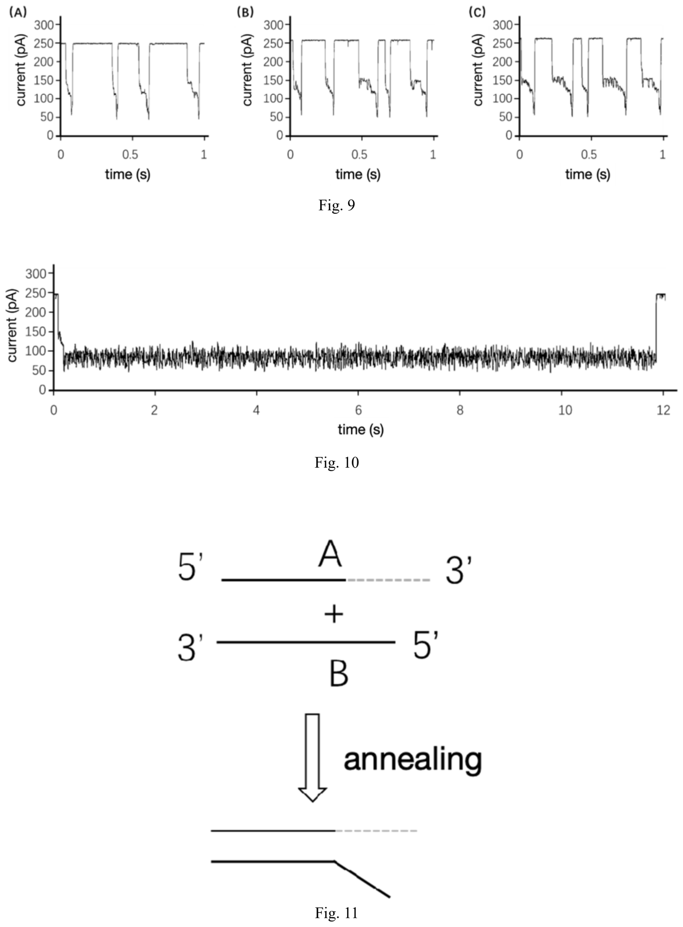

The blocker complexes respectively including blocker-10 iSpC3, blocker-20 iSpC3, and blocker-30 iSpC3 as prepared in step 1.1 "Preparation of blocker Complex", together with the tethering-sequence-1, were introduced into the system. Under a voltage of 180 mV, the current signal profiles of the blocker translocation through the nanopore were observed and obtained. The translocation duration of a single blocker complex was on the order of 100 milliseconds. The buffer was as follows: 470 mM KCl, 25 mM HEPES, 10 mM MgCl₂, 30 mM ATP, pH = 8.10, and the temperature was 30 °C. Fig. 9 shows the current signal profiles of the three blocker complexes translocating through the nanopore.

### 1.4 Nanopore Sequencing of the Library-to-be-Sequenced Using the blocker Complexes and the Tethering Sequence

In accordance with the reference (Ji Z, Guo P. Channel from bacterial virus T7 DNA packaging motor for the differentiation of peptides composed of a mixture of acidic and basic amino acids. Biomaterials. 2019 May 21; 214:119222), a single-channel nanopore detection system was constructed based on a patch-clamp and a signal amplifier, with an embedment of a single pore protein into the membrane.

The blocker complexes respectively including blocker-10 iSpC3, blocker-20 iSpC3, and blocker-30 iSpC3 as prepared in step 1.1 "Preparation of blocker Complex", together with the tethering-sequence-1 and the library-to-be-sequenced-1, were mixed and introduced into the single pore system. With sequencing under a voltage of 180 mV, the current signal profiles of the library during nanopore sequencing were observed and obtained (Fig. 10), indicating that the solution employing the blocker complex and the tethering sequence is feasible for nanopore sequencing.

### Example 2

This Example was conducted with a blocker shown in panel C of Fig. 4 according to the present disclosure, specifically including the following steps.

### 2.1 Preparation of blocker

The following sequences were separately dissolved with TE buffer (pH = 8) in accordance with the manufacturer's instructions.

5'-GAGGCGAGCGGTCAATTTTTTTTTTXXXXXXXXXX-3' (X=iSpC3; SEQ ID NO:10); 5'-GAGGCGAGCGGTCAATTTTTTTTTTXXXXXXXXXXXXXXXXXXXX-3' (X=iSpC3; SEQ ID NO:11); 5'-GAGGCGAGCGGTCAATTTTTTTTTTXXXXXXXXXXXXXXXXXXXXXXXXXXXXXX-3 " (X=iSpC3; SEQ ID NO:12); 5'-AATGCCTAAGTACTGAAATTGACCGCTCGCCTC-3' (SEQ ID NO: 13).

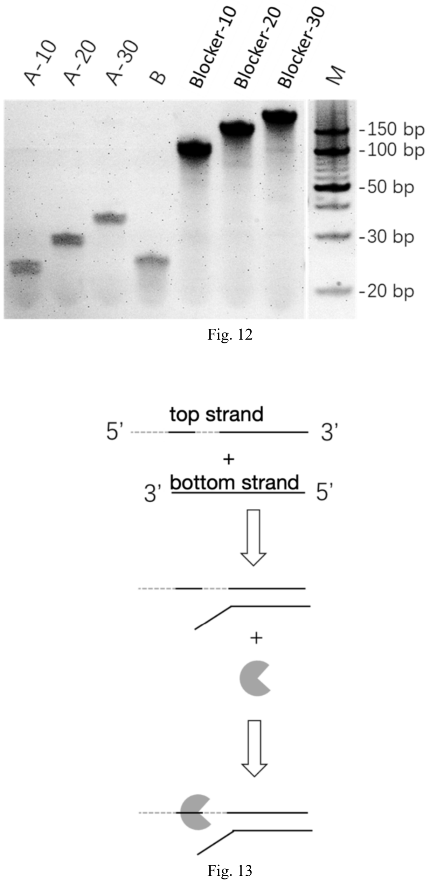

The top strands (SEQ ID NO: 10, SEQ ID NO: 11, SEQ ID NO: 12) were individually subject to anneal with the bottom strand (SEQ ID NO: 13) at a molar ratio of 1:1 to prepare blockers, which were named as blocker-10, blocker-20, and blocker-30, respectively. The annealing procedure is as follows: incubate at 95 °C for 5 minutes, cool down to 25 °C at a rate of 0.1 °C/s, and continue incubation for 30 minutes. The structures of the top and bottom strands of the blocker are shown in Fig. 11, where the top strand A sequentially includes, in the 5' to 3' direction, a bottom strand-complementary sequence (indicated by black full line) and a guide sequence (indicated by gray dashed line); and the bottom strand B sequentially includes, in the 5' to 3' direction, a tethering-sequence-2-complementary sequence and a top strand-complementary sequence (both indicated by black full lines), where the tethering-sequence-2 is 5'-CAGTACTTAGGCATTYYYY-Cholesterol 3' (Y=iSp18; SEQ ID NO: 14). After annealing, electrophoresis detection was performed. The results are shown in Fig. 12, demonstrating that the annealed blockers were successfully prepared, where lanes 1-3 show the top strands A-10, A-20 and A-30 (respectively containing 10, 20, and 30 iSpC3 units) respectively, lane 4 shows the bottom strand B, lanes 5-7 show the annealed blocker-10, blocker-20, and blocker-30 respectively, and lane 8 shows the DNA molecular weight marker (M).

### 2.2 Preparation of Sequencing Library

The following sequences were separately dissolved with TE buffer (pH = 8) in accordance with the manufacturer's instructions.

5'-XXXXXXXXXXXXXXXXXXXXXXXXXXXXXXTTTTTTTTTTYYYYGGTTGTTTCTGTTGGTG CTGATATTGCT-3' (X=iSpC3 , Y=iSp18; SEQ ID NO: 7); 5'-Phosphorylation-GCAATATCAGCACCAACAGAAACAACCTTTGAGGCGAGCGGTCAA-3' (SEQ ID NO: 15).

The sequences of SEQ ID NO: 7 and SEQ ID NO: 15 were subject to anneal at a molar ratio of 1:1 to prepare a sequencing adapter. The annealing procedure is as follows: incubate at 95 °C for 5 minutes, cool down to 25 °C at a rate of 0.1 °C/s, and continue incubation for 30 minutes. The structure of the sequencing adapter is shown in Fig. 13, where the top strand sequentially includes, in the 5' to 3' direction, a guide sequence (30 iSpC3 units, indicated by gray dashed line), a helicase-binding sequence (10 thymine residues, indicated by black full line), a blocking sequence (4 iSp18 units, indicated by gray dashed line), and a bottom strand-complementary sequence (indicated by black full line); and the bottom strand sequentially includes, in the 5' to 3' direction, a top strand-complementary sequence and a blocker-complementary sequence (indicated by black full line).

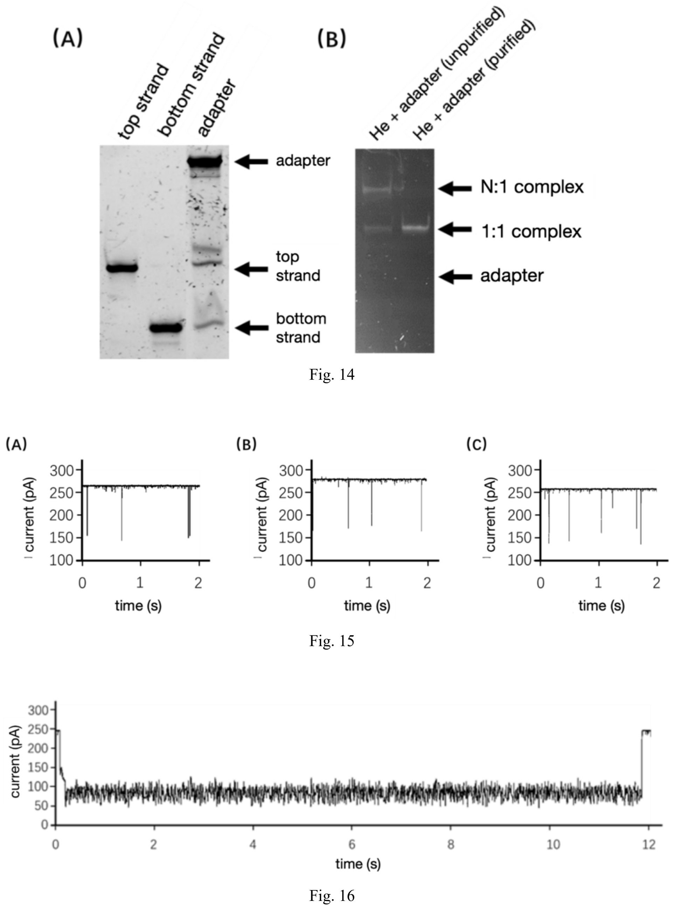

After annealing, electrophoresis detection was performed. The results are shown in part A of Fig. 14, demonstrating that the sequencing adapter was successfully prepared through anneal, where lane 1 shows the top strand (SEQ ID NO: 7), lane 2 shows the bottom strand (SEQ ID NO: 15), and lane 3 shows the sequencing adapter after annealing.

The helicase He (T4Dda-(ΔM1) G1/E94C/C109A/C136A/K194L/A360C; SEQ ID NO: 6) was expressed in prokaryotic cells *Escherichia coli,* and was obtained as a target protein through multi-step purification.

The heliccase and the sequencing adapter were mixed at a molar ratio of 9:1 in a reaction buffer with a final concentration of 25 mM HEPES, 50 mM KCl, 0.5 mM EDTA, and 2.5 mM MgCl₂ (pH = 8.0), and were incubated at room temperature for 30 minutes.

To the incubation product 0.25 volumes of 5 mM ATP was added, and was incubated at room temperature for 30 minutes. Owing to the blocking sequence, only 1 molecule of the helicase He bound between the guide sequence and the blocking sequence was retained.

The sequencing adapter complexes were purified with AMPure XP beads (Beckman Coulter, Cat. No. A63882), to obtain the purified sequencing adapter complexes (refer to Fig. 13, the helicase is represented by the light gray circle with a gap, with a helicase direction of 5' to 3'). The purified sequencing adapter complexes were subject to electrophoresis detection, and the results are shown in part B of Fig. 14, demonstrating that a large quantity of sequencing adapter complexes each with the sequencing adapter and the helicase at a ratio of 1:1, where lanes 1-2 respectively shows the unpurified complex obtained by the incubation of the adapter with the helicase and a purified one derived from the same incubation.

The sequencing adapter complex was ligated to randomly fragmented genomic DNAs of *Bacillus subtilis* by T4 DNA Ligase (NEB, Cat. No. M0202T). With purification via AMPure XP beads, a library-to-be-sequenced-2 was obtained.

### 2.3 Nanopore Sequencing with the blocker

In accordance with the reference (Ji Z, Guo P. Channel from bacterial virus T7 DNA packaging motor for the differentiation of peptides composed of a mixture of acidic and basic amino acids. Biomaterials. 2019 May 21; 214:119222), a single-channel nanopore detection system was constructed based on a patch-clamp and a signal amplifier, with an embedment of a single pore protein into the membrane.

The blocker-10, blocker-20, and blocker-30 as prepared in step 1.1 "Preparation of blocker", together with the tethering-sequence-2 (SEQ ID NO: 14), were individually introduced into the system. Under a voltage of 180 mV, the current signal profiles of the blocker translocation through the nanopore were observed and obtained. The translocation duration of a single blocker was on the millisecond scale. The buffer was as follows: 470 mM KCl, 25 mM HEPES, 10 mM MgCl₂, 30 mM ATP, pH = 8.10, and the temperature was 30 °C. Fig. 15 shows the current signal profiles of the three blockers translocating through the nanopore.

### 2.4 Nanopore Sequencing of the Library-to-be-Sequenced Using the blocker and the Tethering Sequence

In accordance with the reference (Ji Z, Guo P. Channel from bacterial virus T7 DNA packaging motor for the differentiation of peptides composed of a mixture of acidic and basic amino acids. Biomaterials. 2019 May 21; 214:119222), a single-channel nanopore detection system was constructed based on a patch-clamp and a signal amplifier, with an embedment of a single pore protein into the membrane.

The blocker-20 as prepared in step 1.1 "Preparation of blocker", together with the tethering-sequence-2 and the library-to-be-sequenced-2, were mixed and introduced into the single pore system. With sequencing under a voltage of 180 mV, the current signal profiles of the library during nanopore sequencing were observed and obtained (Fig. 16), indicating that the solution employing the blocker and the tethering sequence is feasible for nanopore sequencing.

### Example 3

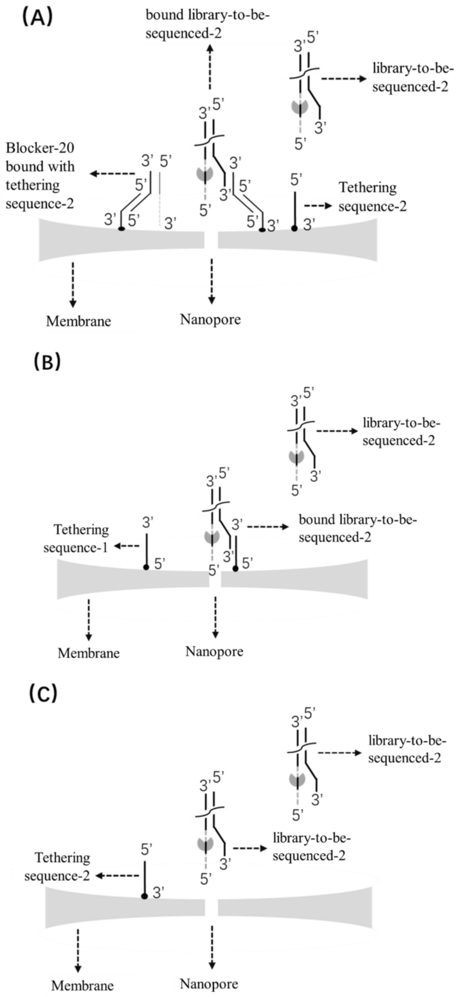

This Example compares read capture yields in nanopore sequencing using "the blocker-20 and tethering sequence-2", "the tethering sequence-1", and "the tethering sequence-2", respectively. Fig. 17 shows a schematic diagram of the experimental design for this Example.

Specifically, in accordance with the reference (Ji Z, Guo P. Channel from bacterial virus T7 DNA packaging motor for the differentiation of peptides composed of a mixture of acidic and basic amino acids. Biomaterials. 2019 May 21; 214:119222), a single-channel nanopore detection system was constructed based on a patch-clamp and a signal amplifier, with an embedment of a single pore protein into the membrane.

For an experimental group, the blocker-20 as prepared in Example 2 and the tethering-sequence-2 were mixed and introduced into the single pore system for sequencing under a voltage of 180 mV for 4 hours. The library-to-be-sequenced-2 (50 ng) was added, performing sequencing under a voltage of 180 mV for 24 hours. Read capture yield was counted every hour after adding the sequencing library. The results showed that as the sequencing time prolonged, the hourly read capture yield first increased and then gradually decreased.

For a control group 1, namely an existing conventional method, the tethering-sequence-1 in Example 1 was introduced into the single pore system for sequencing under a voltage of 180 mV for 4 hours. The library-to-be-sequenced-2 (50 ng) was added, performing sequencing under a voltage of 180 mV for 24 hours. Read capture yield was counted every hour after adding the sequencing library. The results showed that as the sequencing time prolonged, the hourly read capture yield decreased significantly, and the rate of decrease was faster than that of the experimental group. Additionally, the read capture yield of the experimental group was lower than that of this control group 1 in the first 4 hours of sequencing, but surpassed that of this control group 1 afterward.

For a control group 2, the tethering-sequence-2 in Example 2 was introduced into the single pore system for sequencing under a voltage of 180 mV for 4 hours. The library-to-be-sequenced-2 (50 ng) was added, performing sequencing under a voltage of 180 mV for 24 hours. Read capture yield was counted every hour after adding the sequencing library. The results showed that only a small amount of reads were captured during the sequencing, and almost no reads were captured 12 hours after the start of sequencing.

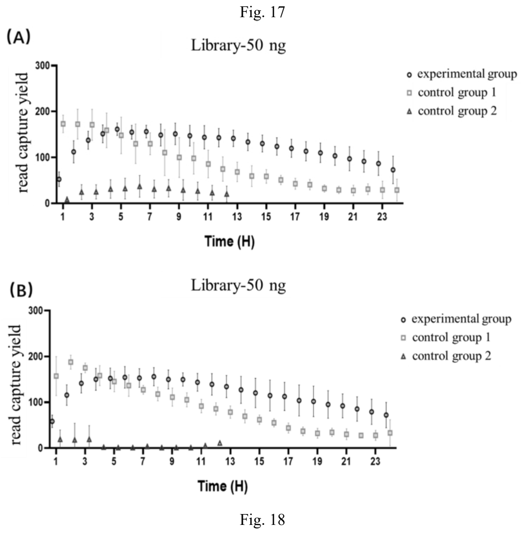

The above experiments were repeated once, and consistent results were obtained (Fig. 18).

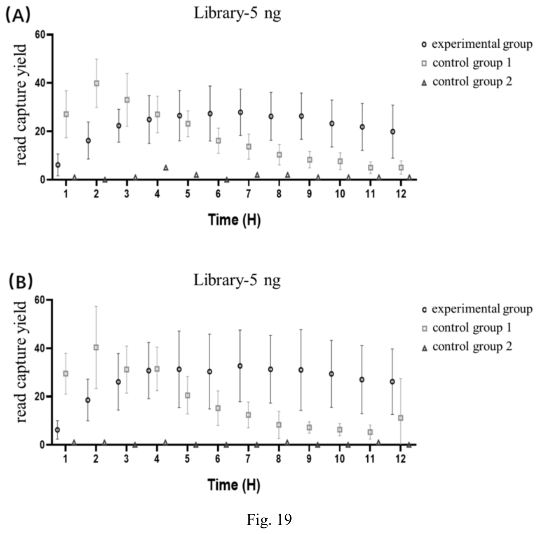

The above experiments were repeated with the library-to-be-sequenced-2 decreased to 5 ng, conducting the sequencing on the experimental group, control group 1, and control group 2, where the sequencing lasted for 12 hours after adding the sequencing library. The experimental results were consistent with those obtained when 50 ng of the sequencing library was used. And such experiments were repeated once, and consistent results were obtained (Fig. 19).

The buffer used in the above experiments was as follows: 470 mM KCl, 25 mM HEPES, 10 mM MgCl₂, 30 mM ATP, pH = 8.10, and the temperature was 30 °C.

With a comparison of the changes in read capture yield over time among the experimental group, control group 1, and control group 2, it is demonstrated that the use of the blocker allows the library to be sequenced to be concentrated within the nanopore capture range, thereby ensuring efficient capture to the library by nanopores during sequencing and improve the utilization efficiency of the library, reflecting tremendous application prospects.

A detailed description of the method for concentrating libraries to be sequenced to be bound within the nanopore capture range using a blocker provided by the present disclosure has been set forth above. Specific Examples have been employed herein to illustrate the principles and modes of practice of the disclosure. It is to be expressly understood that the description of the foregoing Examples is provided solely to aid in the comprehension of the method and core concepts of the present disclosure. Furthermore, based on the concepts of the present disclosure, variations may be made in the specific implementations and fields of application. Accordingly, the specification shall not be construed to limit the present disclosure.

## Claims

1. A nanopore sequencing method, comprising the following steps:
i. incubating a blocker with a membrane embedded with nanopores, such that the blocker is coupled to the membrane, wherein the blocker comprises:
a tethering sequence, comprising a portion coupled to the membrane;
a first strand, comprising a first sequence, and a second sequence at least partially complementary to a second strand; and
the second strand, comprising a third sequence at least partially complementary to the first strand, configured to bind with a sequencing library by means of complementation with a sequencing adapter, and a fourth sequence at least partially complementary to the tethering sequence,
ii. applying an electric field force to the membrane of step i, such that the first strand of the blocker within a nanopore capture range translocates through the nanopore, exposing the third sequence in the second strand of the blocker; and
iii. binding, the third sequence, with the sequencing library via the sequencing adapter, such that the sequencing library is bound within the nanopore capture range and further captured by the nanopore, thereby achieving a sequencing on a strand to be sequenced ligated with the sequencing adapter.

2. The method according to claim 1, wherein the portion coupled to the membrane in the tethering sequence is a hydrophobic molecule,
preferably, the hydrophobic molecule is selected from any one or more of: a lipid, fatty acid, sterol, carbon nanotube, polypeptide, protein and/or amino acid;
more preferably, the hydrophobic molecule is selected from any one or more of: cholesterol, palmitate or tocopherol.

3. The method according to claim 1 or 2, wherein the first sequence consists of 10 to 50 nucleotides, iSpC3, iSp18, or a combination thereof.

4. The method according to any one of claims 1 to 3, wherein the first strand further comprises a nucleic-acid-controlling-protein binding sequence, and a first nucleic-acid-controlling-protein is introduced in step i.

5. The method according to claim 4, wherein the first nucleic-acid-controlling-protein is selected from any one of a helicase, topoisomerase, ligase, or polymerase.

6. The method according to claim 4 or 5, wherein the first nucleic-acid-controlling-protein acts in a direction from 3' to 5'or from 5' to 3'.

7. The method according to any one of claims 4 to 6, wherein the first strand further comprises a blocking sequence, located between the second sequence and the nucleic-acid-controlling-protein binding sequence.

8. The method according to claim 7, wherein the blocking sequence consists of at least one of iSp18, iSp9, iSpC3, iSpC6, and iSpC12.

9. The method according to claim 7 or 8, wherein the blocking sequence has a length of at least 1 bp.

10. The method according to any one of claims 1 to 9, wherein the second sequence, the third sequence, and the fourth sequence each has a length of at least 2 bp,
preferably, the second sequence, the third sequence, and the fourth sequence each has a length of 5 bp to 30 bp.

11. The method according to any one of claims 1 to 10, wherein the third sequence is complementary, in a length of at least 2 bp, to the sequencing adapter of the sequencing library.

12. The method according to any one of claims 1 to 11, wherein the blocker is composed of DNA, RNA, LNA, PNA, BNA, or any combination thereof.

13. The method according to any one of claims 1 to 12, wherein the nanopore is a transmembrane protein pore or a solid-state pore,
preferably, the transmembrane protein pore is selected from hemolysin, MspA, MspB, MspC, MspD, FraC, ClyA, PA63, CsgG, CsgD, XcpQ, SP1, phi29 connector protein, InvG, GspD, or any combination thereof.

14. The method according to any one of claims 1 to 13, wherein the membrane is an amphiphilic membrane, a high-molecular polymer membrane, or any combination thereof.

15. The method according to any one of claims 1 to 14, wherein a voltage applied during sequencing is above 10 mV,
preferably, the voltage is 50 mV to 250 mV.

16. The method according to any one of claims 1 to 15, wherein the sequencing library is introduced into a reaction system simultaneously with, prior to, or subsequent to an introduction of the blocker.

17. The method according to any one of claims 1 to 16, wherein the sequencing adapter consists of two nucleic acid chains, with a side of one nucleic acid chain complementary to a side of the other nucleic acid chain, and another side of one nucleic acid chain complementary to another side of the other nucleic acid chain, the blocker binds to the sequencing adapter in a manner comprises:
a. under the sequencing adapter having a Y-shaped structure, with a side being a non-complementary, single stranded portion and another side being a complementary, double stranded portion, the third sequence of the blocker complementarily binds to the single stranded portion of the sequencing adapter; or
b. under the sequencing adapter having a side being a non-complementary, single stranded portion and another side being a complementary, double stranded portion comprising a RNA fragment, after degrading the RNA fragment with a ribonuclease, the third sequence of the blocker complementarily binds to a complementary sequence of the RNA fragment.

18. The method according to any one of claims 1 to 17, wherein the sequencing adapter binds with a second nucleic-acid-controlling-protein, forming a sequencing adapter complex,
preferably, the second nucleic-acid-controlling-protein is a helicase.

19. The method according to any one of claims 1 to 18, wherein in step iii, the third sequence of the blocker is complementary to the sequencing adapter of the sequencing library, such that the blocker links to the sequencing library; and under an action of the electric field force, the strand to be sequenced in the sequencing library translocates through the nanopore, and a current signal change generated by translocation of the strand to be sequenced through the nanopore is detected, thereby obtaining sequence information of the strand to be sequenced.

20. A nanopore sequencing kit, comprising: a blocker, a sequencing buffer, a sequencing adapter and a second nucleic-acid-controlling-protein, wherein the blocker comprises:
a tethering sequence, comprising a portion coupled to a membrane in the sequencing;
a first strand, comprising a first sequence, and a second sequence at least partially complementary to a second strand; and
the second strand, comprising a third sequence at least partially complementary to the first strand, and a fourth sequence at least partially complementary to the tethering sequence,
wherein the sequencing adapter forms, with the second nucleic-acid-controlling-protein, a sequencing adapter complex to capture a strand to be sequenced, thereby producing a sequencing library, and
the third sequence is configured to bind with the sequencing library complementarily, such that the sequencing library is bound within a nanopore capture range and further captured by the nanopore, to achieve sequencing.

21. The nanopore sequencing kit according to claim 20, wherein the portion coupled to the membrane in the tethering sequence is a hydrophobic molecule,
preferably, the hydrophobic molecule is selected from any one or more of: a lipid, fatty acid, sterol, carbon nanotube, polypeptide, protein and/or amino acid;
more preferably, the hydrophobic molecule is selected from any one or more of: cholesterol, palmitate or tocopherol.

22. The nanopore sequencing kit according to claim 20 or 21, wherein the first sequence consists of 10 to 50 nucleotides, iSpC3, iSp18, or a combination thereof.

23. The nanopore sequencing kit according to any one of claims 20 to 22, wherein the first strand further comprises a nucleic-acid-controlling-protein binding sequence, and the kit further comprises a first nucleic-acid-controlling-protein.

24. The nanopore sequencing kit according to claim 23, wherein the first nucleic-acid-controlling-protein is selected from any one of a helicase, topoisomerase, ligase, or polymerase.

25. The nanopore sequencing kit according to claim 23 or 24, wherein the first nucleic-acid-controlling-protein acts in a direction from 3' to 5'or from 5' to 3'.

26. The nanopore sequencing kit according to any one of claims 20 to 25, wherein the first strand further comprises a blocking sequence, located between the second sequence and the nucleic-acid-controlling-protein binding sequence.

27. The nanopore sequencing kit according to claim 26, wherein the blocking sequence consists of at least one of iSp18, iSp9, iSpC3, iSpC6, and iSpC12.

28. The nanopore sequencing kit according to claim 26 or 27, wherein the blocking sequence has a length of at least 1 bp.

29. The nanopore sequencing kit according to any one of claims 20 to 28, wherein the second sequence, the third sequence, and the fourth sequence each has a length of at least 2 bp,
preferably, the second sequence, the third sequence, and the fourth sequence each has a length of 5 bp to 30 bp.

30. The nanopore sequencing kit according to any one of claims 20 to 29, wherein the third sequence is complementary, in a length of at least 2 bp, to the sequencing adapter of the sequencing library.

31. The nanopore sequencing kit according to any one of claims 20 to 30, wherein the blocker is composed of DNA, RNA, LNA, PNA, BNA, or any combination thereof.
